(19) Europäisches Patentamt
European Patent Office
Office européen des brevets

(11) **EP 2 457 613 A1**

(12) **EUROPÄISCHE PATENTANMELDUNG**

(43) Veröffentlichungstag:
**30.05.2012 Patentblatt 2012/22**

(51) Int Cl.:
***A61N 1/05*** *(2006.01)*

(21) Anmeldenummer: **11189537.1**

(22) Anmeldetag: **17.11.2011**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **24.11.2010 US 416778 P**

(71) Anmelder: **BIOTRONIK SE & Co. KG**
**12359 Berlin (DE)**

(72) Erfinder:
• **Büssing, Heinrich**
**10317 Berlin (DE)**
• **Frenzel, Timo**
**12435 Berlin (DE)**

(74) Vertreter: **Lindner-Vogt, Karin L.**
**Biotronik SE & Co. KG**
**Corporate Intellectual Properties**
**Woermannkehre 1**
**12359 Berlin (DE)**

(54) **Implantierbare Leitung mit einem langgestreckten Leitungskörper**

(57)     Implantierbare Leitung mit einem langgestreckten Leitungskörper und einem sich in Längsrichtung des Leitungskörpers erstreckenden Funktions-Leiter, der zur Realisierung einer medizinischen Funktion der Leitung wirkt, wobei zusätzlich zum Funktions-Leiter und isoliert von diesem in den Leitungskörper eine Mehrzahl von Ringschluss-Leiterelementen eingebettet ist, welche eine Kopplung des Funktions-Leiters mit einem äußeren Wechselmagnetfeld verringern oder den Transport von elektromagnetischer Hochfrequenzenergie entlang der Leitung dämpfen.

**FIG. 1**

Printed by Jouve, 75001 PARIS (FR)

**Beschreibung**

Die Erfindung betrifft eine implantierbare Leitung mit einem langgestreckten Leitungskörper und einem sich in Längsrichtung des Leitungskörpers erstreckenden Funktions-Leiter, der zur Realisierung einer medizinischen Funktion der Leitung wirkt. Derartige Leitungen sind insbesondere Stimulationselektrodenleitungen (mitunter verkürzt auch als "Elektroden" bezeichnet) von Herzschrittmachern oder Schockelektrodenleitungen von implantierbaren Defibrillatoren, es können aber auch Katheter sein, die eine langgestreckte leitfähige Struktur enthalten.

Medizinische Implantate wie die erwähnten Schrittmacher und Defibrillatoren besitzen häufig eine elektrische Verbindung zum Körperinneren des Patienten. Eine solche Verbindung dient zur Messung von elektrischen Signalen oder zur Stimulation von Körperzellen. Diese Verbindung ist oft als längliche Elektrode ausgeführt. Gegenwärtig werden elektrische Signale zwischen dem Implantat und den Elektrodenkontakten (Spitze, Ringe, HV-Schockwendeln, Sensoren o. ä.) mit elektrisch gut leitenden Materialien übertragen.

Wird ein System aus Implantat und Elektrode starken Störfeldern (EMI, MRI) ausgesetzt, kann es zu unerwünschtem Fehlverhalten kommen, speziell einer Erwärmung von Teilen des Systems oder elektrischen Fehlfunktionen (z.B. Resets). Die Erwärmung kann zu Schädigungen von Körpergewebe oder von Organen führen, wenn die erwärmten Teile direkten Kontakt zum Gewebe haben. Dies ist insbesondere bei der Elektrodenspitze der Fall.

Die Ursache für das unerwünschte Fehlverhalten ist die Wechselwirkung des Feldes mit der länglichen Leitungsstruktur der Elektrode: Die Elektrode wirkt als eine Antenne und empfängt Energie aus den umgebenden Feldern. Diese Energie auf den therapeutisch genutzten Leitungen kann die Antenne distal über die Elektrodenkontakte (Spitze, Ring ..) an das Gewebe oder proximal an das Implantat abgeben.

Die gleichen Probleme treten auch bei anderen länglichen leitfähigen Strukturen auf, deren proximales Ende nicht zwingend mit einem Implantat verbunden ist (z.B. bei Kathetern, temporären Elektroden, Stents, etc.).

Bekannt sind abgeschirmte Elektroden. Die Schirmung der Elektrode wirkt vor allem gegen von außen einkoppelnde elektrische Felder. Außerdem sind diese Schirmungen nur mit einer entsprechenden Schirmstärke wirksam und langzeitstabil. Es ist daher ein Kompromiss zu finden zwischen einer Vergrößerung des Elektrodendurchmessers - mit entsprechenden Auswirkungen für die Kosten und die Handlichkeit der Elektrode - und Einbußen in der Schirmwirkung.

Zur Vermeidung von Störungen durch magnetische Wechselfelder und speziell in Magnetresonanz-Apparaturen (MRI), speziell zur Begrenzung von Erwärmungen der Elektrodenspitze in solchen Feldern, wurde in der US 2008/0243218 A1 das Vorsehen eines in Längsrichtung abwechselnd hin und her geführten Schutzleiters in einer Elektrodenleitung vorgeschlagen. Dieses sogenannte Billabong-Prinzip nutzt ebenfalls Gegeninduktivitäten zur Minderung induzierter Ströme. Allerdings ist in diesem Fall durch die dreilagige Wendelwicklung ebenfalls eine Vergrößerung des Elektrodendurchmessers zu erwarten. Zudem wird die Elektrode eine geringere Leitfähigkeit aufweisen.

Aus Ladd M., Quick H.: Reduction of resonant RF heating in intravascular catheters using coaxial chokes, Magnetic Resonance in Medicine, 2000, sind Schutzvorkehrungen gegen durch HF-Resonanzen bewirkte Erwärmungen intravaskulärer Katheter in Form äußerer Schutzdrosseln (sog. Chokes) bekannt. Derartige Chokes liegen an der Außenhülle der Elektrode und wirken gegen Oberflächenströme. Diese Lösung reduziert keine Ströme, die in die Innenwendel einkoppeln. Außerdem ist eine Vergrößerung des Elektrodendurchmessers zu erwarten, mit den o. g. Folgen.

Der Erfindung liegt die Aufgabe zugrunde, eine verbesserte implantierbare Leitung der genannten Art bereit zu stellen, die in starken äußeren elektromagnetischen Wechselfeldem verbesserte Eigenschaften aufweist und konstruktiv leicht und somit kostengünstig realisierbar ist.

Diese Aufgabe wird durch eine implantierbare Leitung mit den Merkmalen des Anspruchs 1 gelöst. Zweckmäßige Fortbildungen des Erfindungsgedankens sind Gegenstand der abhängigen Ansprüche.

Ein wesentlicher Gedanke der Erfindung besteht darin, den Einfluss starker äußerer Felder durch das Einbetten einer Mehrzahl zusätzlicher leitfähiger Elemente in der implantierbaren Leitung zu reduzieren. Die zusätzlichen, zum Funktionsleiter isolierten Leiter (hier Ringschluss- oder auch Feldentkopplungs-Letierlemente genannt), die insbesondere als lokale Gegeninduktivitäten wirken, verändern die Wechselwirkung zwischen dem äußeren Feld und der Leitung so, dass sich auf der Leitung eine andere Stromverteilung ausbildet. Sie verringern eine Kopplung des Funktions-Leiters mit einem äußeren Wechselmagnetfeld bzw. erhöhen die Dämpfung der entlang der Leitung transportierten elektrischen Hochfrequenzenergie. Die ungewollten Antenneneigenschaften der Leitung verändern sich durch diese Verstimmung. Dies resultiert in einer geringeren Erwärmung der distalen Leitungskontakte. Dieser Vorteil gilt für verschiedene geometrische Formen und verschiedene Lagen der Leitung.

Die Ringschluss-Leiterelemente können dabei beispielsweise eine Nickel-KobaltLegierung, insbesondere MP35N, aufweisen.

In einer Ausführung der Erfindung sind die Ringschluss-Leiterelemente *(Anmerkung: ins Englische soll es übersetzt werden als: „ circuit-elements " oder evtl. " inductive resistive circuit elements ")* als in Längsrichtung des Leitungskörpers beabstandet von einander gereihte Ringe ausgebildet. Derartige kleine Ringe sind sehr kostengünstig verfügbar. In alternativen Ausführungen sind die Feldentkopplungs-Leiterelemente als in Längsrichtung des Leitungskörpers beabstandet von einander gereihte Drahtschleifen oder -wicklungen ausgebildet. Die erwähnten, eine Gegeninduktivität bildenden Ringe können auch aus leitend miteinander verbundenen Ring-Segmenten zusammengesetzt sein.

In einer weiteren Ausführung der Erfindung ist vorgesehen, dass die Feldentkopplungs-Leiterelemente über die Länge des Leitungskörpers gleichmäßig verteilt, insbesondere mit gleichen Abständen zueinander angeordnet sind. Hierbei sind insbesondere die Abstände zwischen den Funktions-Leiterelementen kleiner als ihr Durchmesser. Eine solche Reihung der Feldentkopplungs-Leiterelemente mit relativ kleinem Abstand sichert eine durchgehende Wirkung der induktiven Feldentkopplung über die gesamte Länge und alle praktisch denkbaren Biegungszustände der Leitung.

In einer weiteren Ausführung ist vorgesehen, dass die Feldentkopplungs-Leiterelemente in vorgeformte Ausnehmungen des Leitungskörpers eingelegt sind. Entsprechende Nuten lassen sich relativ leicht in den Kunststoff- bzw. Silikon-Leitungskörper einformen und sichern, dass die Leiterelemente ihre gleichmäßige Reihung auch bei relativ starken mechanischen Beanspruchungen - etwa im Zuge der Implantation oder einer etwaigen Repositionierung - bewahren.

In einer praktisch besonders bedeutsamen Ausführung der erfindungsgemäßen Leitung weist diese einen ersten und zweiten koaxial zueinander verlaufenden Funktions-Leiter auf. Hierbei sind die Feldentkopplungs-Leiterelemente in radialer Richtung zwischen dem ersten und zweiten Funktions-Leiter, vom ersten oder zweiten Funktions-Leiter isoliert, angeordnet.

Während bei den vorgenannten Ausführungen einzelne kurze Feldentkopplungs-Leiter zum Schutz des Funktionsleiters bzw. der Funktionsleiter zum Einsatz kommen, ist in anderen Ausführungen auch der Einsatz relativ langgestreckter Leiterelemente denkbar. So umfassen in einer weiteren Ausführung die Feldentkopplungs-Leiterelemente eine sich in Längsrichtung des Leitungskörpers erstreckende Wendel, welche ein Teilabschnitt des oben dargestellten gewendelten Funktions-Leiters ist, deren Windungen keinen Kontakt zueinander haben. Die isoliert verlaufenden Windungen sind über die Länge des Leitungskörpers durch elektrisch verbindende Längsdrähte, die sich in Richtung der Längsachse des Leitungskörpers erstrecken, miteinander verbunden. Eine Modifikation der zuletzt genannten Ausführung sieht so aus, dass als Feldentkopplungs-Leiterelemente die isoliert verlaufenden Windungen der erwähnten Wendel des Funktions-Leiters vorgesehen ist, welche über mindestens einen geneigt zur Längsrichtung der Leitungskörpers verlaufenden Verbindungsdraht erfolgt. Dieser erstreckt sich mindestens über einen Teilabschnitt des Leitungskörpers und verbindet mindestens zwei benachbarte Windungen der Wendel. Gemäß einer weiterführenden Ausgestaltung sind die Verbindungsdrähte Teilabschnitte einer gegenläufig zur Wendel des Funktions-Leiters verlaufenden Drahtwendel.

Die Wendel des Funktionsleiters kann vorzugsweise als Band oder Draht ausgelegt sein.

Vorteile und Zweckmäßigkeiten der Erfindung ergeben sich im Übrigen aus der nachfolgenden Beschreibung von speziellen Ausführungen anhand der Figuren. Von diesen zeigen:

Fig. 1                       eine Prinzipskizze zur Erläuterung der Erfindung,

Fig. 2A und 2B       Prinzipdarstellungen einer Ausführungsform der erfindungsgemäßen implantierbaren Leitung, in Längsrichtung (Fig. 2A) und in Art einer Querschnittsdarstellung (Fig. 2B),

Fig. 3A bis 3C        Prinzipskizzen einer weiteren Ausführungsform der erfindungsgemäßen Leitung und

Fig. 4A bis 4C        Prinzipskizzen einer weiteren, gegenüber Fig. 3A bis 3C modifizierten Ausführung, sowie

Fig. 5                       eine schematische Längsschnittdarstellung eines Abschnitts einer weiteren erfindungsgemäßen Leitung und

Fig. 6                       eine schematische Längsschnittdarstellung eines Abschnitts einer weiteren erfindungsgemäßen Leitung.

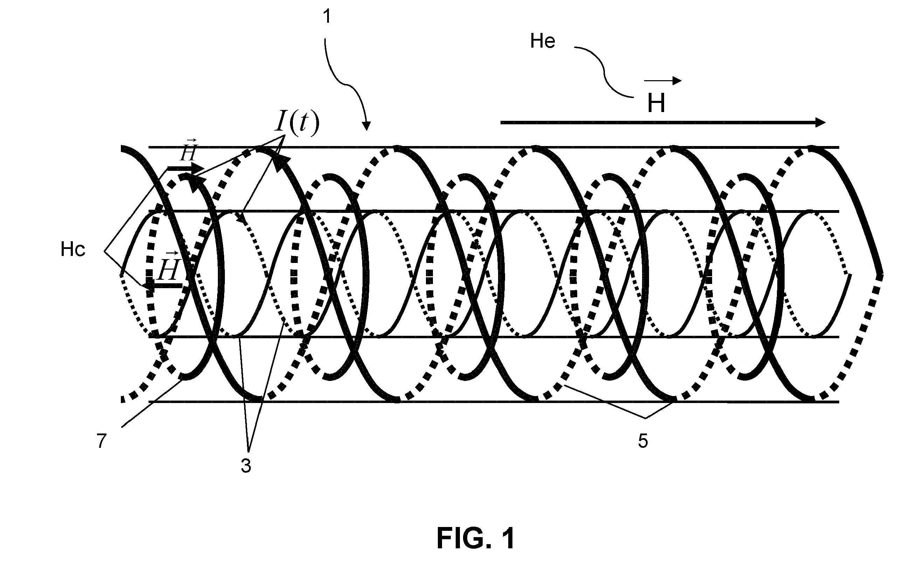

Fig. 1 ist eine Prinzipskizze zur Erläuterung der Erfindung, die nur die leitfähigen Elemente einer Elektrodenleitung 1 als Ausführungsbeispiel einer implantierbaren Leitung, nicht aber deren isolierenden Leitungskörper, zeigt. Bei der dargestellten Ausführung sind ein aus jeweils mehreren Drähten ineinander gewickelter Innenleiter 3 (zusammengefasst auch bezeichnet als „erster Funktions-Leiter") und ein ebenfalls aus mehreren ineinander gewickelten Drähten gebildeter Außenleiter 5 („zweiter Funktions-Leiter") vorgesehen. Innen-und Außenleiter sind bei dieser Ausführung gegensinnig gewickelt. Sie können im Gebrauch einem externen Wechselmagnetfeld $H_e$ ausgesetzt sein, welches in den Leitern einen Stromfluss I(t) induziert.

Zur Verringerung nachteiliger Einflüsse dieser Induktionsströme sind im Zwischenraum zwischen Innen- und Außenleiter (erstem und zweitem Funktions-Leiter) mit gleichen Abständen zueinander leitfähige Ringe (auch zu bezeichnen als Induktionsringe bzw. Feldentkopplungs-Leiterelemente) 7 angeordnet. Diese erzeugen aufgrund des in ihnen bewirkten zeitabhängigen Stromflusses und elektrischen Widerstandes ein Kompensations-Magnetfeld $H_c$, welches die Wirkung des externen Wechselmagnetfeldes $H_e$ wenigstens teilweise kompensiert oder elektrische Verluste, die den Transport von elektrischer Energie entlang der Leitung mindern.

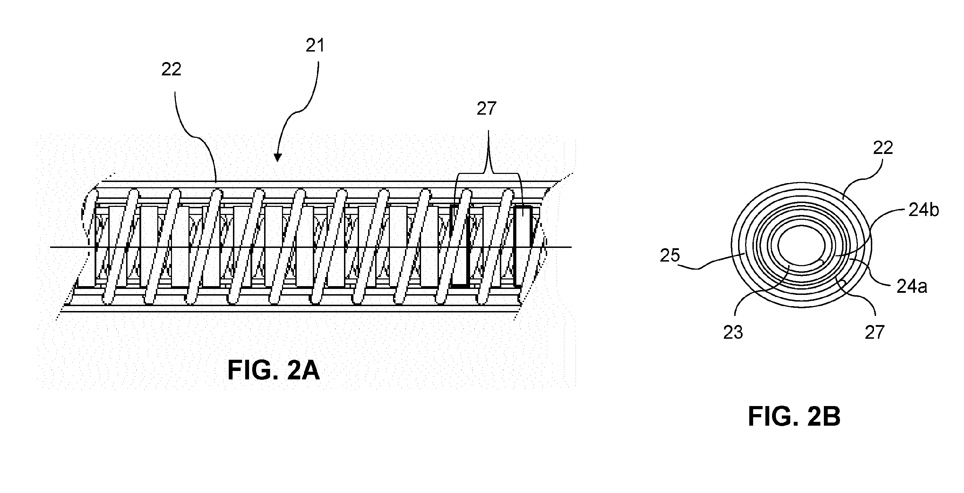

Fig. 2A und 2B zeigen etwas genauer die konstruktive Ausführung einer erfindungsgemä-βen Leitung 21, wobei der Prinzipskizze in Fig. 1 entsprechende Elemente mit an jene Figur angelehnten Bezugsziffern bezeichnet sind. Die Leitung 21 umfasst, eingebettet in einen (hier schematisch dargestellten) Leitungskörper 22 und voneinander jeweils isoliert durch eine innere Isolierhülle 24a und mittlere Isolierhülle 24b, eine Innenwendel 23 — die hier wieder mehrere ineinander gewickelte Wendeln umfasst -, eine Außenwendel 25 sowie zwischen diesen angeordnete Induktionsringe 27. Obwohl diese Figuren nicht als maßstäbliche Darstellungen zu versehen sind, soll Fig. 2B verdeutlichen, dass die Dicke der Induktionsringe 27 wesentlich geringer als diejenige der Funktions-Leiter 23 und 25 ist.

Hinsichtlich der Geometrie der Wendeln bzw. Funktions-Leiter einerseits und der Induktionsringe oder —hülsen andererseits erfolgt eine Erläuterung anhand einer speziellen Elektrodenleitung, bekannt als Setrox-Elektrode. Diese umfasst eine Wendel aus vier Drähten von 0.13mm Durchmesser. Der mittlere Durchmesser $d_i$ der Wendel ist 0.57mm. In der Wendel soll der Strom $I_i$ fließen, und zwar ein Wechselstrom der Kreisfrequenz $\omega$. Als Windung ist eine Wicklung aller vier Drähte bezeichnet. Der Gangunterschied einer solchen Windung ist $\approx$0,13mm.4.105% = 0,546mm bei $\approx$5% Kompression. Ganz außen mit dem mittleren Durchmesser ist eine Hülse z.B. aus MP35N vorgesehen, auf der der Strom $I_a$ fließt, der von der Innenwendel induziert wird. Diese Hülse ist gegen die Innenwendel elektrisch isoliert. Der Stromfluss kommt dadurch zustande, dass die Hülse die Fläche der Innenwendel mit umschließt und die beiden somit induktiv gekoppelt sind.

Die Induktion, die vom Strom der Innenwendel verursacht wird und nur in der Fläche der Innenwendel zu finden ist, ist $B_i = \mu \dfrac{I_i \cdot N}{l}$. Dabei ist N die Anzahl der Windungen, die entlang der Strecke $l$ liegen, die dicht an dicht mit Hülsen überzogen ist, $l \gg d_a$. Die Induktion, die vom Strom auf der Hülse verursacht wird, ist $B_a = \mu \dfrac{I_a}{l}$. Um den magnetischen Fluss zu bekommen, der durch die Hülse geht, sind die Induktionen mit den Flächeninhalten zu multiplizieren, die ihre verursachenden Ströme einschließen.

$$\Phi_{ges} = \Phi_i + \Phi_a = \frac{\pi \cdot d_i^2}{4} \cdot \mu \cdot \frac{I_i \cdot N}{l} + \frac{\pi \cdot d_a^2}{4} \cdot \mu \cdot \frac{I_a}{l} = L_i \cdot I_i + L_a \cdot I_a \quad \text{mit} \quad L_i := \frac{\mu \cdot \pi \cdot N \cdot d_i^2}{4 \cdot l} \quad \text{und}$$

$$L_a := \frac{\mu \cdot \pi \cdot d_a^2}{4 \cdot l}.$$

Die Hülse ist ein geschlossener Stromkreis, so dass sich für die Summe der Spannungen null ergibt. Die Spannungen auf der Hülse setzen sich aus der Induktionsspannung von der Innenwendel mit $j\omega I_i L_i$, der Selbstinduktion der Hülse mit $j\omega I_a L_a$ und dem Spannungsabfall über dem Widerstand der Hülse $I_i R_a$ zusammen. Somit muss $j\omega I_i L_i + J\omega I_a L_a + I_a R_a = 0$ sein und es ergibt sich für den induzierten Strom in der Hülse $I_a = \dfrac{j\omega L_i}{R_a + j\omega L_a} I_i$.

Um die Wirkung der Hülsen oder induktiven Ringe auf die Innenwendel zu erhalten muss die Rückwirkung des induzierten Stroms $I_a$ auf die Innenwendel untersucht werden. Die gleiche Maschengleichung, wie für die Hülse, wird nun also für die Innenwendel aufgestellt, wobei $U_i$ die Spannung ist, die an den Enden der Elektrodenleitung, bzw. Wendel, angeschlossen ist.

$$U_i = I_i R_i + j\omega I_i L_i N + j\omega \frac{\frac{\pi}{4} d_i^2}{\frac{\pi}{4} d_a^2} N L_a I_a = I_i R_i + j\omega I_i L_i N + j\omega \frac{d_i^2}{d_a^2} N L_a \frac{j\omega L_i}{R_a + j\omega L_a} I_i$$

Es ist hierbei berücksichtigt, dass vom magnetischen Fluss $\Phi_a$, den die Hülse erzeugt, nur ein Teil $\dfrac{\frac{\pi}{4} d_i^2}{\frac{\pi}{4} d_a^2} \Phi_a$

auch durch die Fläche $\frac{\pi}{4}d_i^2$ der Wendel dringt. Außerdem geht der mag-netische Fluss durch alle Windungen der Wendel und muss daher mit $N$ malgenommen werden. Trennung von Real- und Imaginärteil, bzw. Wirk- und Blindwiderstand, führen zu dem Ausdruck für die Impedanz der Wendel

$$Z_i = \frac{U_i}{I_i} = R_i + \frac{\omega^2 N L_i L_a}{R_a^2 + \omega^2 L_a^2}\frac{d_i^2}{d_a^2} + j\omega L_i N\left(1 - \frac{d_i^2}{d_a^2}\frac{\omega^2 L_a^2}{R_a^2 + \omega^2 L_a^2}\right)$$

Wenn man diese insgesamt durch die Länge 1 teilt und somit $Z_i^{'}$, $L_i^{'} = N'L_i$ und R; als Widerstände und Induktivitäten pro Längeneinheit auffasst erhält man abschließend

$$Z_i' = R_i' + \frac{\omega^2 L_i' L_a}{R_a^2 + \omega^2 L_a^2}\frac{d_i^2}{d_a^2} + j\omega L_i'\left(1 - \frac{d_i^2}{d_a^2}\frac{\omega^2 L_a^2}{R_a^2 + \omega^2 L_a^2}\right).$$

Zum Widerstand der Innenwendel bei Gleichstrom $R_i^{'}$ gesellt sich also noch ein frequenz-abhängiger Teil

$$\frac{\omega^2 L_i' L_a}{R_a^2 + \omega^2 L_a^2}\frac{d_i^2}{d_a^2}$$ hinzu

Die Wellenzahl $k$ entlang der Leitung richtet sich nach Induktivitäts-, Kapazitäts- und Widerstandsbelägen, $k^2 = \omega^2 C'L' - G'R' - j\omega(C'R' + L'G')$, wobei G' der Leitwert des Isolationsschlauchs ist und näherungsweise, als perfekter Isolator angenommen, zu null gesetzt wird. Es bleibt somit $k = \sqrt{\omega^2 C'L' - j\omega C'R'} =: \beta + j\alpha$, wobeit β der Realteil der Wellenzahl ist und die Wellenlänge $\left(\lambda = \frac{2\pi}{\beta}\right)$ beschreibt, wohingegen α die Dämpfungskonstante der Welle auf der Leitung ist. $\alpha = \sqrt{\dfrac{\sqrt{(\omega^2 L'C')^2 + (\omega R'C')^2} - \omega^2 L'C'}{2}}$ falls $\omega^2 C'L' > \omega C'R'$. Wenn man hier aus der Formel für die Impedanz der Wendel $Z_i^{'}$ die Werte für $R' = R_i' + \frac{\omega^2 L_i' L_a}{R_a^2 + \omega^2 L_a^2}\frac{d_i^2}{d_a^2}$ und

$$L' = L_i'\left(1 - \frac{d_i^2}{d_a^2}\frac{\omega^2 L_a^2}{R_a^2 + \omega^2 L_a^2}\right)$$ einsetzt, erhält man den optimalen Wert $R_{a'opt}$, der die höchste Dämpfung ergibt mit $R_{a'opt} \approx \omega L_a$.

Für eine vierfache Innenwendel mit 0,57 mm mittlerem Durchmesser und 0,13 mm Drahtdurchmesser ergibt sich L'≈1,07 μH/m. Realistischerweise könnten darüber Hülsen mit einem mittleren Durchmesser von $d_a$ =0,77 mm geschoben werden. Wenn diese aus MP35N wären, wäre die optimale Wandstärke 10,63 μm. In dem Fall, dass die Abstände der Hülsen genauso lang sind wie die Hülsen, muss die Wandstärke der Hülsen verdoppelt werden, damit man auf den Meter Elektrodenlänge gemittelt wieder auf den optimalen Widerstandswert kommt.

Außerdem sollen die Abstände der Ringe oder Hülsen voneinander wesentlich kleiner sein, als ihr Durchmesser $d_a$. Diese als Hülsen beschriebenen Bauteile können auch in Form von geschlossenen Drahtschleifen vorliegen. Hauptsache ist, dass sie sich dicht an dicht reihen und geschlossene Schleifen mit optimalem Widerstand darstellen, um starke Dämpfung der Hochfrequenzwelle zu erzielen.

Bei 10,63 $\mu$m Wandstärke der Hülsen würde sich in diesem Beispiel zum Widerstand des Drahtes der Wendel von 66 $\Omega$/m dann bei 64 MHz noch ein Widerstand von 220 $\Omega$/m addieren. Die Induktivität der Wendel wäre dann bei 64 MHz nur noch 0,777 $\mu$H/m. Mit einem Kapazitätsbelag von 160 pF/m ergibt sich ohne Ringe eine Dämpfungskonstante $\alpha_{ohne}$=0,402 Np/m und mit Ringen $\alpha_{mit}$=1,88 Np/m. Nimmt man an, dass entlang der Elektrode gleichmäßig elektrische Energie eingekoppelt und zur Elektrodenspitze weitergeleitet wird, so würde insbesondere die Energie, die dicht am proximalen Ende in die Wendel fällt zum distalen Ende hin stärker bedämpft werden.

Es zeigt sich, dass unter diesen Bedingungen eine Verringerung des Stroms um 30% und eine Verringerung der Energie in der Spitze um 50% bei einer 60 cm langen Elektrode möglich ist.

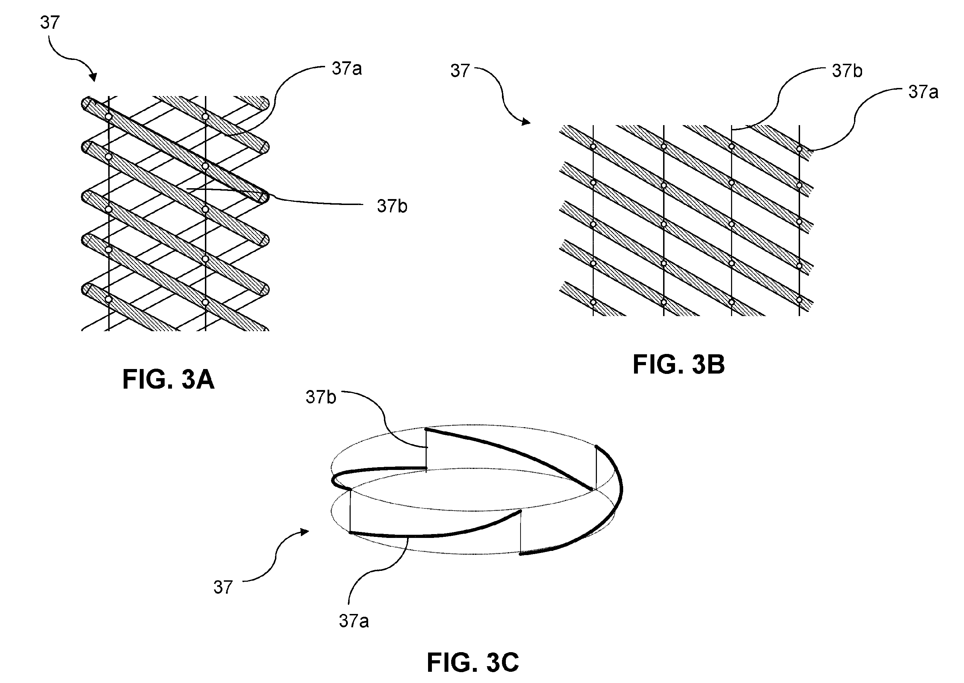

Fig. 3A bis 3C zeigen skizzenartig in Art einer Seitenansicht (Fig. 3A) bzw. einer Abwicklung auf die Ebene (Fig. 3B) bzw. einer perspektivischen Ausschnittdarstellung (Fig. 3C) eine weitere Ausführung der Ringschluss-Leiterelemente 7 einer erfindungsgemäßen Leitung. Hier umfassen die Ringschluss-Leiterelemente 37 ein spiralig gewendeltes Band bzw. einen Draht 37a, welches bzw. welcher ein Teilabschnitt des oben dargestellten gewendelten Funktions-Leiters ist, und die einzelnen Wicklungen der Wendeln 37a in Längsrichtung der Leitung leitend miteinander verbindende Verbindungsdrähte 37b. Die Verbindungsdrähte 37b können an die Wendel 37a angeschweißt oder auch leitfähig angeklebt oder angelötet sein.

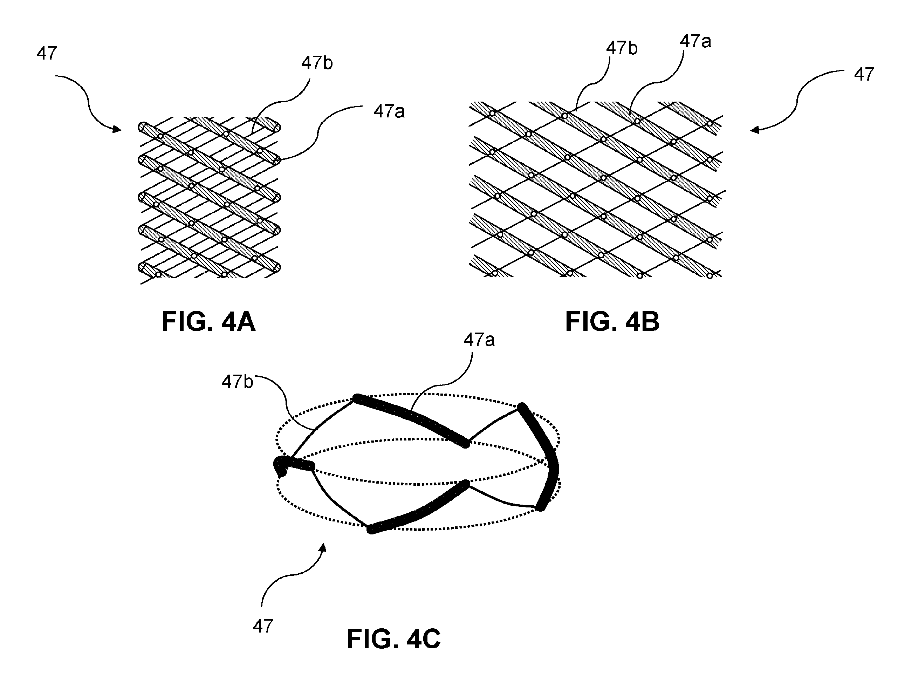

Die Figuren 4a bis 4C zeigen in einer der Darstellung nach Fig. 3A bis 3C entsprechenden Weise als modifizierte Ausführung Feldentkopplungs-Leiterelemente 7 und 47, die wiederum eine Band- oder Drahtwendel 47a und Verbindungsdrähte 47b wie soeben beschrieben umfassen. Anders als bei der vorgenannten Ausführungsform verlaufen die Verbindungsdrähte hier aber nicht in Längsrichtung der Leitung, sondern hierzu geneigt. Dies erleichtert wesentlich Verformungen der Leitung, bei denen die geneigten Drähte dann lokal ihren Neigungswinkel ändern, während längsverlaufende Verbindungsdrähte einer Verformung einen erheblichen Widerstand entgegensetzen. Um die Verformung weiter zu erleichtern, kann es auch vorgesehen werden, dass die Wendel, welche die Verbindungsdrähte bildet, anders als oben beschrieben nicht angeschweißt oder auch leitfähig angeklebt oder angelötet ist, sondern dass die Wendel mit den Verbindungsdrähten auf die Funktionsleiterwendel "aufgecrimpt" ist, beispielsweise dadurch, dass der Innendurchmesser beider Wendeln identisch ist und die Wendel mit den Verbindungswendeln außen auf die Funktionsleiterwendel aufgespannt ist.

Die Figuren 3C bzw. 4C zeigen jeweils, wie der durch das externe Wechselmagnetfeld im Ringschluss-Leiterelement induzierte Strom in einem Segment über jeweils vier Viertelwindungen der Wendel 37a bzw. 47a und die Verbindungsdrähte 37b 47b zu einem Ringschluss nimmt. Da der Ringschluss (bzw. der hierdurch gebildete integrierte induktive Ring) sich mit der zugehörigen Elektrodenwendel (also dem Funktions-Leiter) fast die gleiche eingeschlossen Fläche teil, ist die induktive Kopplung größer als bei den weiter oben erläuterten in Fig. 1 und 2A und 2B gezeigtem Induktionsringen bzw. —hülsen.

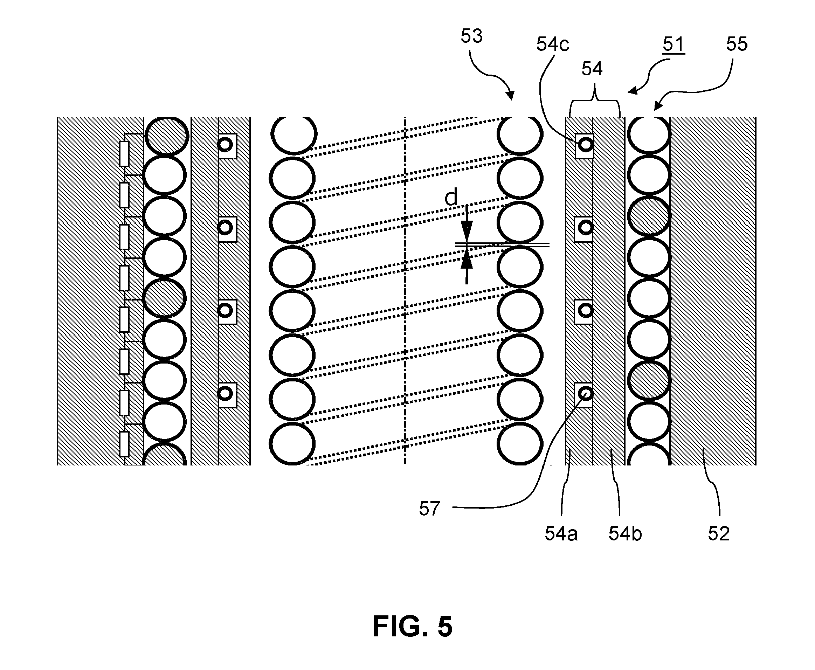

Fig. 5 zeigt in einer schematischen Querschnittsdarstellung als weiteres Ausführungsbeispiel eine erfindungsgemäße Leitung 51, bei der in einen Leitungskörper 52 eine aus zwei Einzeldrähten gewickelte Innenwendel 53 und eine aus vier Einzeldrähten gewickelte Au-βenwendel 55 eingebettet ist. Zwischen Innen- und Außenwendel ist eine aus zwei konzentrischen Zylindern 54a, 54b zusammengesetzte Isolierung 54 vorgesehen. Der innere Isolierstoff 54a dieser Isolierung hat auf der Außenseite eingeformte Nuten bzw. Rillen 54c, in die Drahtringe 57 als Induktionsringe im Sinne der Prinzipsskizze nach Fig. 1 eingelegt sind. Das Durchmesser-Verhältnis zwischen diesen Drahtringen 55 und den Drähten der Innen- und Außenwendel 53, 55 soll verdeutlichen, dass der Drahtdurchmesser der Induktionsringe wesentlich geringer als derjenige der Funktions-Leiter ist.

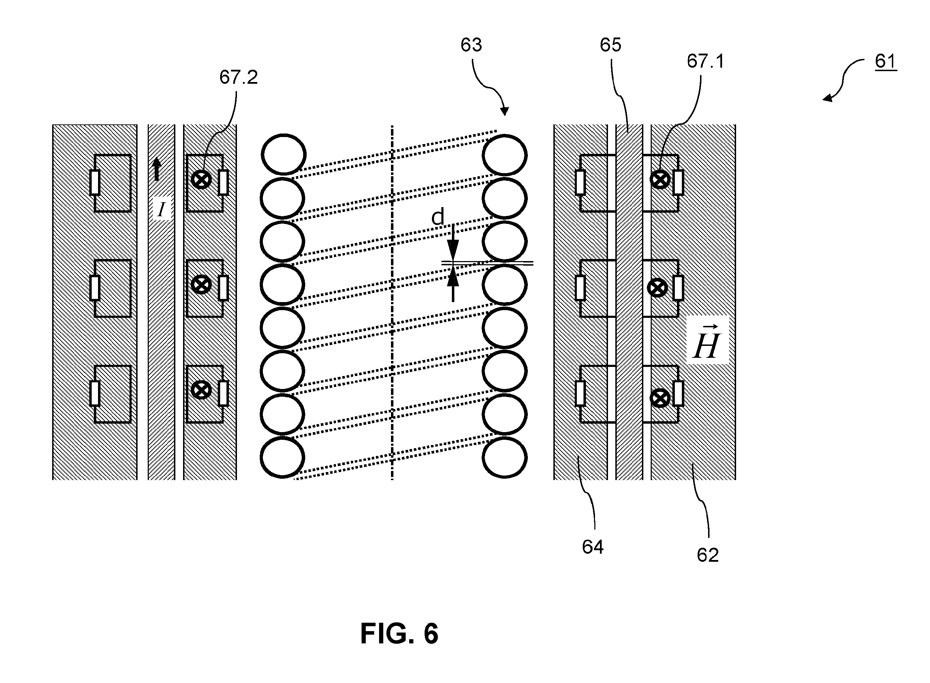

Fig. 6 zeigt in einer synergistischen Darstellung als abgewandelte Ausführung(en) eine weitere Elektrodenleitung 61 mit einem Leitungskörper 62 und einer mittleren Isolierung 64, deren Innenleiter-Struktur 63 der Ausführung nach Fig. 5 entspricht, die aber statt einer Vierfach-Wendel als Außenleiter eine Seilstruktur 65 hat. Auf der rechten Seite der Figur sind Feldentkopplungs-Ringe (Induktionsringe) 67. 1 dargestellt, die in die Innenoberfläche des äußeren Leitungskörpers 62 derart eingelegt sind, dass sie einen - widerstandsbehafteten — elektrischen Kontakt mit dem Außenleiter 65 haben, während auf der linken Seite der Figur symbolisch Ringschluss-Leiterelemente 67.2 dargestellt sind, die in die mittlere Isolierung 64 ohne elektrischen Kontakt mit dem Außenleiter 65 eingebettet sind.

In beiden Figuren 5 und 6 steht das Symbol eines elektrischen Widerstands für einen Draht mit Widerstandsbelag, und als Material des Leitungskörpers und der Zwischen-Isolierung ist jeweils Silikon angenommen. Die in beiden Figuren gezeigten konkreten Abmessungen sind lediglich beispielhaft als Angaben für marktgängige Elektrodenleitungen zu verstehen, die mit den erfindungsgemäßen Mitteln verbessert werden sollen.

Die Ausführung der Erfindung ist nicht auf die oben beschriebenen Beispiele und hervorgehobenen Aspekte beschränkt, sondern ebenso in einer Vielzahl von Abwandlungen möglich, die im Rahmen fachgemäßen Handelns liegen.

**Patentansprüche**

1. Implantierbare Leitung mit einem langgestreckten Leitungskörper und einem sich in Längsrichtung des Leitungskörpers erstreckenden Funktions-Leiter, der zur Realisierung einer medizinischen Funktion der Leitung wirkt, wobei zusätzlich zum Funktions-Leiter und isoliert von diesem in den Leitungskörper eine Mehrzahl von Ringschluss-Leiterelementen eingebettet ist, welche eine Kopplung des Funktions-Leiters mit einem äußeren Wechselmagnetfeld verringern oder die Dämpfung der entlang der Leitung transportierten elektrischen Hochfrequenzenergie erhöhen.

2. Leitung nach Anspruch 1, wobei die Ringschluss-Leiterelemente als in Längsrichtung des Leitungskörpers beabstandet von einander gereihte Ringe oder RingSegmente ausgebildet sind.

3. Leitung nach Anspruch 2, wobei die Ringschluss-Leiterelemente als in Längsrichtung des Leitungskörpers beabstandet von einander gereihte Drahtschleifen oder - wicklungen ausgebildet sind.

4. Leitung nach Anspruch 1, wobei die Ringschluss-Leiterelemente über die Länge des Leitungskörpers gleichmäßig verteilt, insbesondere mit gleichen Abständen zueinander angeordnet sind.

5. Leitung nach Anspruch 4, wobei die Abstände zwischen dem Ringsschluss-Leiterelementen kleiner als ihre Durchmesser sind.

6. Leitung nach Anspruch 1, wobei die Ringschluss-Leiterelemente in vorgeformte Ausnehmungen des Leitungskörpers eingelegt sind.

7. Leitung nach Anspruch 1, welche einen ersten und zweiten, koaxial zueinander verlaufenden Funktions-Leiter aufweist, wobei die Ringschluss-Leiterelemente in radialer Richtung zwischen dem ersten und zweiten Funktions-Leiter, von ersten oder zweiten Funktions-Leiter isoliert, angeordnet sind.

8. Leitung nach Anspruch 1, wobei als Ringschluss-Leiterelemente eine sich in Längsrichtung des Leitungskörpers erstreckende Wendel des Funktions-Leiters, deren Windungen keinen Kontakt zueinander haben, und die Windungen über die Länge des Leitungskörpers elektrisch miteinander verbindende Längsdrähte, die sich in Richtung der Längsachse des Leitungskörpers erstrecken, vorgesehen sind.

9. Leitung nach einem der Ansprüche 8, wobei die Wendel des Funktions-Leiters als Band oder Draht ausgelegt ist.

10. Leitung nach Anspruch 1, wobei als Ringschluss-Leiterelemente eine sich in Längsrichtung des Leitungskörpers erstreckende Wendel des Funktions-Leiters, deren Windungen keinen Kontakt zueinander haben, und geneigt zur Längsachse des Leitungskörpers über jeweils mindestens einen Teilabschnitt seines Umfangs verlaufende Verbindungsdrähte zur elektrischen Verbindung mindestens zweier benachbarter Windungen Wendel vorgesehen sind.

11. Leitung nach Anspruch 10, wobei die Verbindungsdrähte Teilabschnitte einer gegenläufig zur Wendel des Funktions-Leiters verlaufenden Drahtwendel sind

12. Leitung nach Anspruch 10, wobei die Wendel des Funktions-Leiters als Band oder Draht ausgelegt ist.

13. Leitung nach Anspruch 1, wobei die Feldentkopplungs-Leiterelemente eine Nickel-Kobalt-Legierung, insbesondere MP35N, aufweisen.

**FIG. 1**

EP 2 457 613 A1

FIG. 2B

FIG. 2A

FIG. 3A

FIG. 3B

FIG. 3C

FIG. 4A

FIG. 4B

FIG. 4C

**FIG. 5**

EP 2 457 613 A1

**FIG. 6**

EP 2 457 613 A1

**Europäisches Patentamt**
**European Patent Office**
**Office européen des brevets**

## EUROPÄISCHER RECHERCHENBERICHT

Nummer der Anmeldung

EP 11 18 9537

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 2 110 154 A1 (BIOTRONIK CRM PATENT AG [CH]) 21. Oktober 2009 (2009-10-21)<br>* Ansprüche 1, 8, 9, 12 *<br>* Abbildungen 1A-C, 6, 7, 12 *<br>* Absatz [0008] - Absatz [0016] *<br>* Absatz [0029] - Absatz [0035] *<br>----- | 1,3,4,6,7 | INV.<br>A61N1/05 |
| X | US 2009/149920 A1 (LI YINGBO [US] ET AL) 11. Juni 2009 (2009-06-11)<br>* Abbildungen 12, 15-17 *<br>* Absatz [0056] - Absatz [0075] *<br>----- | 1-7 | |
| X | WO 03/063954 A1 (MEDTRONIC INC [US]) 7. August 2003 (2003-08-07)<br>* Abbildungen 4, 5, 6a, 6b *<br>* Seite 8, Zeile 3 - Seite 12, Zeile 27 *<br>----- | 1,3,4,13 | |
| X | WO 2004/095385 A2 (BIOPHAN TECHNOLOGIES INC [US]; WEINER MICHAEL L [US]; MILLER VICTOR [U) 4. November 2004 (2004-11-04)<br>* Abbildungen 2,32 *<br>* Seite 13, Zeile 22 - Seite 16, Zeile 15 *<br>* Seite 20, Zeile 25 - Seite 26, Zeile 31 *<br>----- | 1,2 | **RECHERCHIERTE SACHGEBIETE (IPC)**<br><br>A61N |

Der vorliegende Recherchenbericht wurde für alle Patentansprüche erstellt

| Recherchenort | Abschlußdatum der Recherche | Prüfer |
|---|---|---|
| München | 7. März 2012 | Ließmann, Frank |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 11 18 9537

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-03-2012

| Im Recherchenbericht angeführtes Patentdokument | Datum der Veröffentlichung | Mitglied(er) der Patentfamilie | Datum der Veröffentlichung |
|---|---|---|---|
| EP 2110154 A1 | 21-10-2009 | EP 2110154 A1<br>US 2009259281 A1 | 21-10-2009<br>15-10-2009 |
| US 2009149920 A1 | 11-06-2009 | EP 2355890 A1<br>US 2009149920 A1<br>WO 2010065049 A1 | 17-08-2011<br>11-06-2009<br>10-06-2010 |
| WO 03063954 A1 | 07-08-2003 | CA 2472264 A1<br>DE 60310194 T2<br>EP 1469910 A1<br>JP 4326340 B2<br>JP 2005515854 A<br>WO 03063954 A1 | 07-08-2003<br>20-09-2007<br>27-10-2004<br>02-09-2009<br>02-06-2005<br>07-08-2003 |
| WO 2004095385 A2 | 04-11-2004 | EP 1622677 A2<br>WO 2004095385 A2 | 08-02-2006<br>04-11-2004 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

• US 20080243218 A1

**In der Beschreibung aufgeführte Nicht-Patentliteratur**

• **LADD M. ; QUICK H.** Reduction of resonant RF heating in intravascular catheters using coaxial chokes. *Magnetic Resonance in Medicine,* 2000